# EUROPEAN PATENT SPECIFICATION

(11) **EP 3 238 641 B1**
(45) Date of publication and mention of the grant of the patent: **06.04.2022**
(21) Application number: 15872466.6
(22) Date of filing: 27.10.2015
(51) Int. Cl.: A61B 17/22, A61M 25/09, A61B 17/28, A61B 17/00

(54) **MEDICAL INSTRUMENT**
MEDIZINISCHES INSTRUMENT
INSTRUMENT MÉDICAL

(30) Priority: 25.12.2014 JP 2014263293
(43) Date of publication of application: 01.11.2017
(73) Proprietor: Olympus Corporation, Hachioji-shi, Tokyo 192-8507 (JP)
(72) Inventor: YOKOTA, Takuo, Hachioji-shi, Tokyo 192-8507 (JP); SATO, Masatoshi, Hachioji-shi, Tokyo 192-8507 (JP); MATSUNAGA, Rei, Hachioji-shi, Tokyo 192-8507 (JP)
(74) Representative: Schicker, Silvia
(86) International application number: PCT/JP2015/080205
(87) International publication number: WO 2016/103897

(56) References cited:
- EP-A2- 2 613 712
- WO-A1-2013/071938
- WO-A1-2013/122690
- JP-A- H10 272 139
- JP-A- 2008 011 970
- US-A- 5 499 991
- US-A- 5 653 716
- US-A1- 2014 222 033
- US-A1- 2014 358 088
- US-B1- 7 651 503
- None

## Description

### Technical Field

The present invention relates to a medical instrument.

Priority is claimed on Japanese Patent Application No. 2014-263293, filed December 25, 2014.

### Background Art

In treatment or inspection of a luminal organ of a human body, it is known to introduce a medical instrument into the luminal organ using a guide wire. When an obstacle such as a stenosis or an occlusion occurs at an opening portion of the luminal organ, the guide wire itself may not be inserted into the luminal organ. For example, when a duodenal papilla is tightly closed, it is difficult to insert the guide wire into a desired luminal organ such as a bile duct or a pancreatic duct via the duodenal papilla.

As a treatment method in such a case, a method called a rendezvous method is known. In the rendezvous method, a guide wire introduced into a bile duct or a pancreatic duct from a body part other than a duodenal papilla is protruded from the duodenal papilla, and an end of the protruding guide wire is held by a medical instrument. The guide wire protruding from the duodenal papilla into a duodenum is pulled to an outside of the body through a treatment tool channel of an endoscope inserted into the duodenum. A stent placement operation or the like is performed using the guide wire pulled to the outside of the body.

When a treatment tool such as a stent is placed by the rendezvous method, the treatment tool is pushed into the bile duct or the pancreatic duct from a papilla through an endoscopic channel similarly to a procedure of ordinary endoscopic retrograde cholangiopancreatography (ERCP) or the like. However, depending on an anatomical structure of a patient, there is a case in which the duodenal papilla may not be seen from a front side in an endoscopic image or the duodenal papilla may be closed tightly. Further, there is a case in which a running state of the bile duct imagined by a surgeon may be different from a real one. In such a case, even if the surgeon attempts to push the treatment tool into the duodenal papilla by an operation at hand, the treatment tool is bent in a space between a distal end of the endoscope and the papilla and the pushing force is easily lost, and thus the treatment tool is not easily introduced.

Therefore, a method of introducing a medical instrument holding a guide wire into the bile duct or the pancreatic duct by retracting the guide wire, which is protruded from the duodenal papilla into the duodenum, into the bile duct or the pancreatic duct instead of pulling the guide wire to the outside of the body through the treatment tool channel has been proposed. A grasping forceps (for example, see Patent Literature 1) is known as the medical instrument for holding the guide wire.

Patent Literature 2 discloses a surgical instrument for passing and/or retrieving a suture through a tissue, the surgical instrument including a needle having a suture slot provided with a distal ramp, a vertical wall, and a wire relief slot. The suture may be clamped or pinned across the suture slot.

Patent Literature 3 discloses a suture passer comprising a hollow tube having a distal end terminating in a sharp point and a proximal end terminating in a handle, with a lumen extending therebetween. The hollow tube is further provided with a radially extending window, communicating with the lumen and configured to selectively receive a suture therein.

Patent Literature 4 discloses a guiding aid comprising a tubular hollow sheath having an operation unit provided at its distal end side. An operation rod is inserted into an inner cavity of the sheath and is provided with a gripping member, formed at its distal end side. The gripping member includes a hook-shaped holding part forming a concave grip surface for holding a target member.

Patent Literature 5 discloses a suture passer including an elongate member configured to pierce a living tissue and defining a central lumen, the passer including a beveled distal end region forming a distal opening having a proximal edge and a distal edge that are formed so as to be sufficiently sharp to pierce a surgical wall. The elongate member has an annular surface forming an acute angle of about 15° to about 30° with a longitudinal axis of the elongated tube. Patent Literature 6 discloses a suture manipulating instrument having a hollow member provided with an elongated needle terminating with a distal end. The elongated needle is configured to curve in the upward direction and defines a passageway terminating with an opening. The elongated needle further defines a longitudinal suture slot which terminates at its distal end with the distal opening.

### Citation List

### Patent Literature

Patent Literature 1: Japanese Unexamined Patent Application, First Publication No. 2008-289556
Patent Literature 2: U.S. Patent Application Publication No. 2014/222033 A1
Patent Literature 3: European Patent Application Publication No. 2 613 712 A2
Patent Literature 4: Japanese Patent Application Publication No. H10 272139 A
Patent Literature 5: International Patent Application Publication No. 2013/122690 A
Patent Literature 6: U.S. Patent Application Publication No. 5 653 716 A

### Summary of Invention

The invention is defined by the independent claim and other embodiments are listed in the dependent claims.

### Technical Problem

In the above-described method of introducing the medical instrument into the bile duct or the pancreatic duct by retracting the guide wire, there are the following problems when the medical instrument to be introduced is the aforementioned grasping forceps.

In general, when the guide wire is grasped by the grasping forceps, the grasping forceps and the guide wire are approximately perpendicular to each other. Even if the guide wire is retracted in this state, a direction of the grasping forceps does not coincide with that of the opening portion, and the grasping forceps does not easily enter the opening portion. Additionally, it is more difficult for the grasping forceps to enter the opening portion if there is an obstacle in the opening portion. It is not impossible to grasp the guide wire by making the grasping forceps and the guide wire become as parallel as possible. However, an advanced technique is required, and thus the number of users having the technique is limited.

The present invention is made in view of such problems and an object of the present invention is to provide a medical instrument which can be appropriately introduced into a luminal organ by a rendezvous method.

### Solution to Problem

According to a first aspect of the present invention, a medical instrument, which is capable of holding a wire includes the features of claim 1. A medical instrument may include: a sheath which has a center axis and includes a first contact area capable of being in contact with the wire and a second contact area capable of being in contact with the wire and provided on a proximal end side beyond the first contact area; and a holding portion which is disposed to be advanceable and retractable in a lumen of the sheath and holds the wire. When the wire is disposed to be in contact with the first contact area and the second contact area, the center axis and the wire between the first contact area and the second contact area form an acute angle at a side of the first contact area.

According to the present invention, the holding portion may be capable of holding the wire on a straight line connecting the first contact area and the second contact area when the sheath is seen from a center axis direction thereof.

An inclined surface which is inclined with respect to the center axis is formed at a distal end of the sheath. The first contact area and the second contact area are provided at the inclined surface.

According to the present invention, a cut-out portion which is cut out along the center axis may be formed in a distal end of the sheath. The first contact area may be provided at a distal end surface of the sheath. The second contact area may be provided at a bottom surface of the cut-out portion.

According to the present invention, a cut-out portion which is cut out along the center axis may be formed in the distal end of the sheath. The other of the first contact area and the second contact area may be provided at a bottom surface of the cut-out portion.

According to the present invention, the holding portion includes an annular portion having a slit portion in which the wire is capable of being inserted, and a plate-shaped portion which extends in a direction of the center axis and is connected to the annular portion.

According to a comparative example, the holding portion may be a two-legged forceps having one pair of grasping portions of which distal ends are disposed to be spaced apart from each other.

According to the present invention, the wire may be a medical guide wire.

According to a further comparative example, the medical instrument may further include a lumen which is formed along the lumen of the sheath and is different from the lumen of the sheath.

### Advantageous Effects of Invention

According to the above-described aspects, the medical instrument can be appropriately introduced into the luminal organ by the rendezvous method.

### Brief Description of Drawings

Fig. 1 is an overall view showing a medical instrument according to a first comparative example.
Fig. 2 is an overall view showing a state in which the medical instrument of Fig. 1 holds a guide wire.
Fig. 3 is a schematic diagram in a cross section of the medical instrument of Fig. 2 taken along a longitudinal direction thereof.
Fig. 4 is a schematic diagram when the medical instrument of Fig. 1 is seen from a center axis direction thereof.
Fig. 5 is an explanatory diagram showing a method of introducing the medical instrument according to the first comparative example.
Fig. 6 is an explanatory diagram showing the method of introducing the medical instrument according to the first comparative example.
Fig. 7 is an explanatory diagram showing the method of introducing the medical instrument according to the first comparative example.
Fig. 8 is an explanatory diagram showing the method of introducing the medical instrument according to the first comparative example.
Fig. 9 is an explanatory diagram showing the method of introducing the medical instrument according to the first comparative example.
Fig. 10 is an explanatory diagram showing the method of introducing the medical instrument according to the first comparative example.
Fig. 11 is an explanatory diagram showing the method of introducing the medical instrument according to the first comparative example.
Fig. 12 is an explanatory diagram showing the method of introducing the medical instrument according to the first comparative example.
Fig. 13 is a diagram showing a modified example of a sheath of the medical instrument according to the first comparative example.
Fig. 14 is an overall view showing a medical instrument of a second comparative example.
Fig. 15 is an overall view showing a state where the medical instrument of Fig. 14 holds the guide wire.
Fig. 16 is a schematic diagram in a cross section of the medical instrument of Fig. 15 taken along a longitudinal direction thereof.
Fig. 17 is a diagram showing a modified example of a cut-out portion of the medical instrument according to the second comparative example.
Fig. 18 is a diagram showing a modified example of the cut-out portion of the medical instrument according to the second comparative example.
Fig. 19 is an overall view showing a medical instrument according to an embodiment of the present invention.
Fig. 20 is an overall view showing a state where the medical instrument of Fig. 17 holds the guide wire.
Fig. 21 is an overall view showing a modified example of a holding portion according to a third comparative example.
Fig. 22 is an overall view showing a modified example of a medical instrument according to a fourth comparative example.
Fig. 23 is an overall view showing a state where the medical instrument of Fig. 20 holds the guide wire. The comparative examples as well as the surgical method steps described below only represent background that is useful for understanding the present invention, but do not form part of the same.

### Description of Embodiment and comparative examples

### [First comparative example]

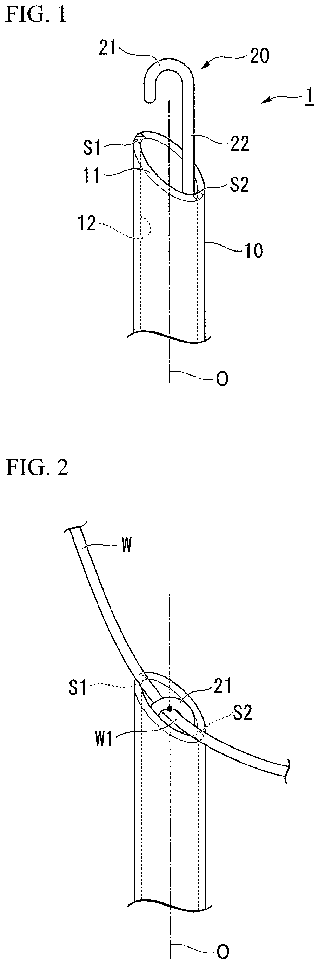

A medical instrument according to a first comparative example will be described with reference to Figs. 1 to 13.

A medical instrument 1 is a medical instrument which is able to hold a wire, for example, a medical guide wire used while being inserted into a human body. The medical instrument 1 includes, as shown in Fig. 1, a sheath 10 and a hook member (a holding portion) 20.

The sheath 10 is formed in a tubular shape having a center axis O and includes a first contact area S1 which can come into contact with a guide wire to be described below and a second contact area S2 which is disposed on a proximal end side beyond the first contact area S1 and can come into contact with the guide wire. Since the sheath 10 is formed by obliquely cutting a distal end of a cylindrical tube, a distal end opening of the sheath 10 is formed in an elliptical shape. The first contact area S1 is an area on the most distal end side of a distal end surface 11 of the sheath 10, and the second contact area S2 is an area on the most proximal end side of the distal end surface 11 of the sheath 10. That is, the first contact area S1 and the second contact area S2 are disposed at positions at which phases thereof are deviated by 180° when being seen from a direction of the center axis O.

The hook member 20 is disposed in a lumen 12 of the sheath 10 to be advanceable and retractable in a direction parallel to the center axis O. Specifically, the hook member 20 includes a hook portion 21 which is formed at a distal end side thereof and a bar-shaped portion 22 which extends toward a proximal end side thereof. The hook portion 21 is configured such that the guide wire can be hooked on and held by the hook portion 21.

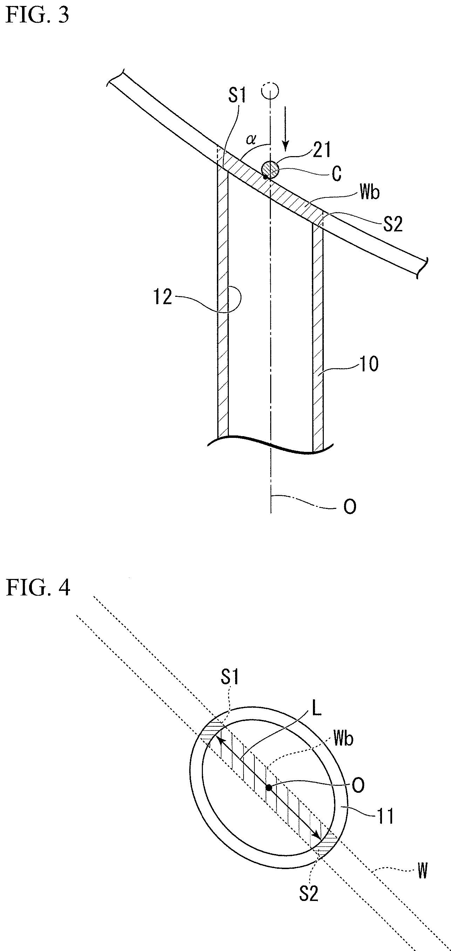

As shown in Fig. 2, when the hook member 20 is slightly retracted while the guide wire W is hooked with the hook portion 21, the guide wire W is pressed to the distal end surface of the sheath 10. As a result, the guide wire W is disposed to be in contact with the first contact area S1 and the second contact area S2. At this time, as shown in the cross section in Fig. 3, the center axis O and a guide wire Wb between the first contact area S1 and the second contact area S2 form an acute angle at a side of the first contact area S1. That is, an angle α formed between the center axis O and the guide wire Wb located on the distal end side beyond a contact portion C at which the hook portion 21 and the guide wire Wb are in contact with each other is an acute angle.

The hook member 20 can hold the guide wire Wb on a straight line L connecting the first contact area S1 with the second contact area S2 when the sheath 10 is seen from the direction of the center axis O, as shown in Fig. 4. Accordingly, the guide wire W can be kept in a state in which the guide wire W is in contact with the first contact area S1 and the second contact area S2.

Next an exemplary method not forming part of the invention of introducing the medical instrument 1 into a luminal organ, for example, a bile duct by a rendezvous method will be described.

As the rendezvous method, there are two methods including a method of puncturing a bile duct (a first luminal organ) from an inside of the body and a method of puncturing the bile duct from an outside of the body. However, in the following description, an example of puncturing the bile duct Bd from the inside of the body is used.

### [First Step]

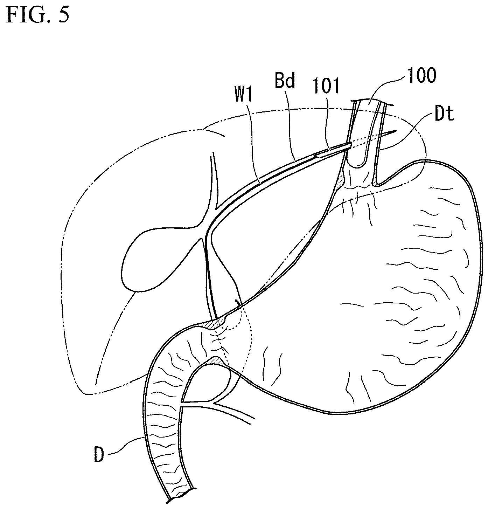

In the case of puncturing the bile duct from the inside of the body, a known ultrasound endoscope is used. First, as shown in Fig. 5, an ultrasound endoscope 100 is inserted orally into an alimentary canal Dt. Then, the bile duct Bd is checked with an ultrasound image, a puncture needle 101 is inserted into the bile duct Bd through the alimentary canal Dt, and a guide wire W1 is protruded from the puncture needle 101 and then introduced into the bile duct Bd.

### [Second Step]

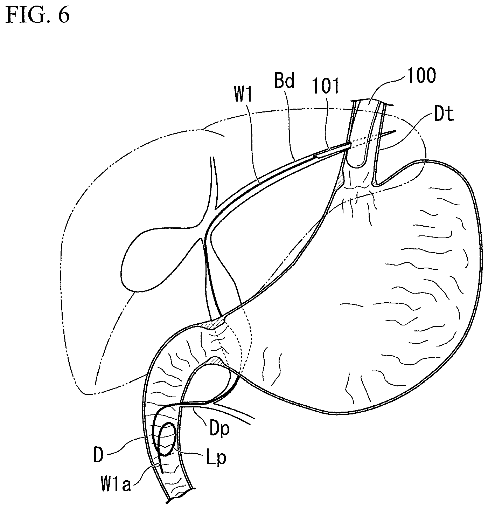

Next, as shown in Fig. 6, a surgeon pushes the first guide wire W1 inserted into the bile duct Bd forward so that a distal end W1a of the first guide wire W1 is protruded from a duodenal papilla (an opening portion) Dp into a duodenum (a second luminal organ) D. By pushing the first guide wire W1 toward the duodenal papilla Dp, the distal end W1a of the first guide wire W1 protruding from the duodenal papilla Dp generally forms a loop Lp in a lumen of the duodenum D. Then, the ultrasound endoscope 100 is removed to the outside of the body, and the distal end W1a of the first guide wire W1 is retained in the duodenum D. At this time, a proximal end side of the first guide wire W1 is located outside the patient's body.

Due to the loop Lp formed at the distal end of the first guide wire W1, the first guide wire W1 can be prevented from coming out of the body together with the ultrasound endoscope 100 when the ultrasound endoscope 100 is removed to the outside of the body. In this manner, the distal end W1a of the first guide wire W1 is retained in the duodenum D.

### [Third Step]

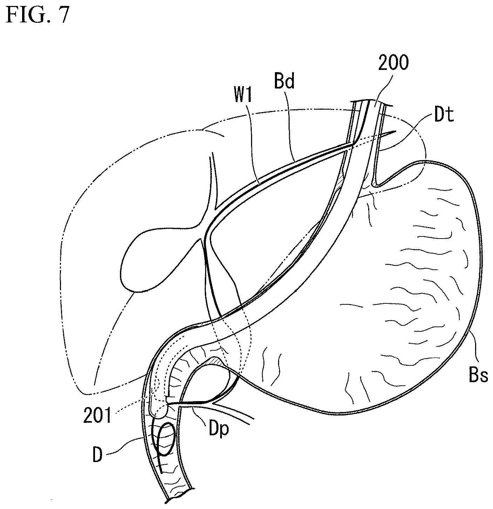

Next, as shown in Fig. 7, a side view type endoscope 200 is inserted to the vicinity of the duodenal papilla Dp via a stomach Bs. Then, the medical instrument 1 is inserted through a treatment tool channel 201 of the endoscope 200.

### [Fourth Step]

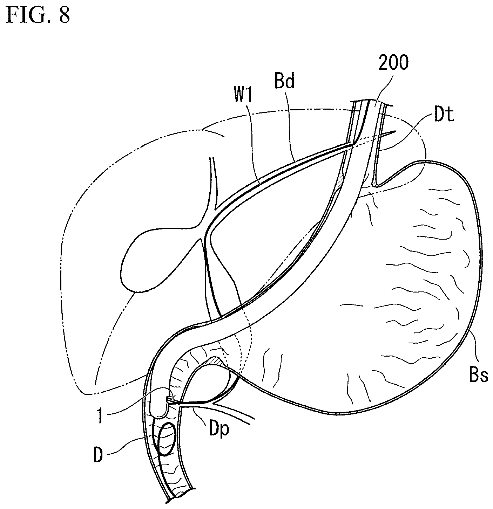

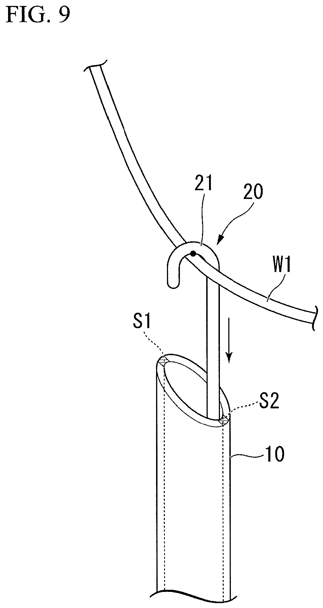

Next, as shown in Fig. 8, the hook member 20 is protruded from the sheath 10 of the medical instrument 1 while the first guide wire W1 protruding from the duodenal papilla Dp is checked with an image of the endoscope 200. Then, as shown in Fig. 9, the first guide wire W1 is hooked with the hook portion 21 of the medical instrument 1 from a side substantially perpendicular thereto. As shown in Fig. 2, the first guide wire W1 is brought into contact with the first contact area S1 and the second contact area S2 by pulling the hook member 20 into the lumen 12 of the sheath 10. The angle α formed by the wire Wb and the center axis O is an acute angle, and the entire guide wire W1 and the medical instrument 1 also form an approximate acute angle.

### [Fifth Step]

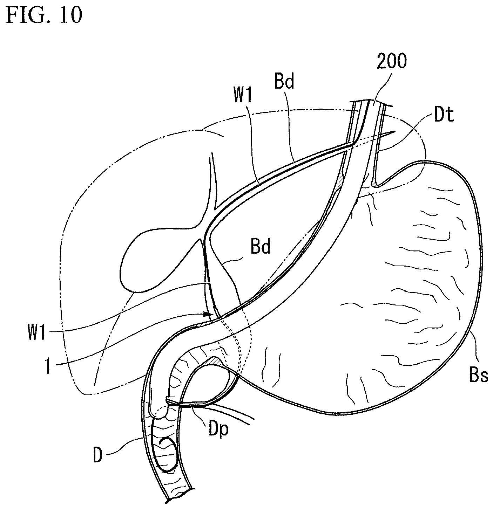

Next, the surgeon pulls the proximal end side of the first guide wire W1 outside the patient's body toward the outside of the body. As a result of this operation, as shown in Fig. 10, the medical instrument 1 is pulled into the bile duct Bd from the duodenal papilla Dp together with the first guide wire W1 while the hook member 20 of the medical instrument 1 holds the first guide wire W1. Since the guide wire W1 and the medical instrument 1 form the acute angle, the guide wire W1 and the medical instrument 1 smoothly enter the bile duct Bd from the duodenal papilla Dp. In accordance with the pulling of the first guide wire W1, the distal end of the medical instrument 1 advances toward an upstream side inside the bile duct Bd. Even at this time, as shown in Fig. 3, since the angle α formed between the center axis O and the wire Wb located on the distal end side beyond the contact area C at which the hook portion 21 and the wire Wb are in contact with each other is the acute angle, the sheath 10 is bent along an extension direction of the guide wire W. In this state, the medical instrument 1 advances inside the bile duct Bd.

### [Sixth Step]

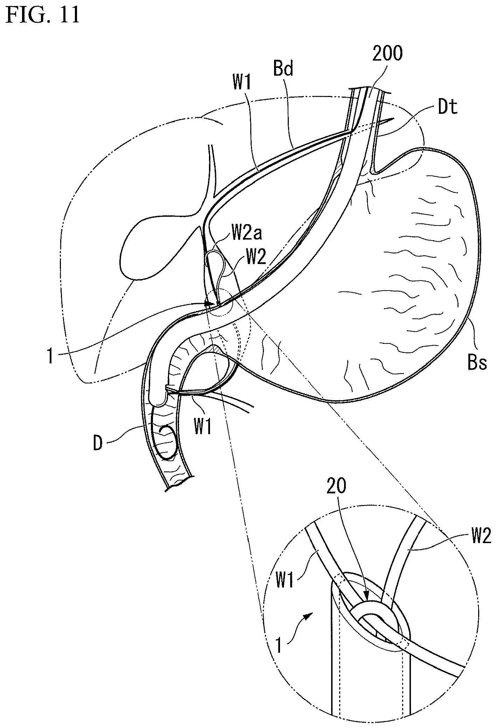

Next, the surgeon inserts a second guide wire W2 from a hand side into the sheath 10 inserted through the treatment tool channel 201 of the endoscope 200 so that the second guide wire W2 is protruded from the distal end of the sheath 10. Accordingly, as shown in Fig. 11, the second guide wire W2 is placed in the bile duct Bd from the distal end opening of the sheath 10. A distal end W2a of the second guide wire W2 also forms a loop like the first guide wire W1. At this time, a proximal end side of the second guide wire W2 is located outside the patient's body.

### [Seventh Step]

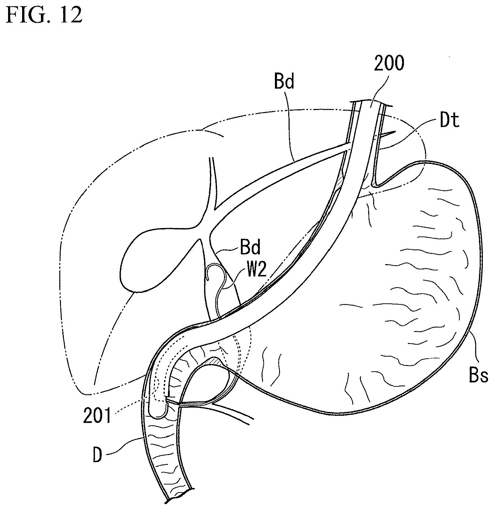

Next, the surgeon releases the holding of the first guide wire W1 by the medical instrument 1 and pulls the proximal end side of the first guide wire W1 toward the outside of the body so that the first guide wire W1 is removed to the outside of the body. Further, as shown in Fig. 12, the surgeon retracts the medical instrument 1 so that the medical instrument 1 is accommodated in the treatment tool channel 201 of the endoscope 200 and then removes the medical instrument 1 to the outside of the body through the treatment tool channel 201.

### [Eighth Step]

Next, the same treatment as that in ordinary endoscopic retrograde cholangiopancreatography (ERCP) is performed using the second guide wire W2. Since a procedure of ERCP is the same as the known procedure, a description thereof will be omitted.

According to the medical instrument 1 of the present comparative example, since the sheath 10 is formed such that the center axis O and the guide wire Wb between the first contact area S1 and the second contact area S2 form the acute angle at the side of the first contact area S1 when the guide wire W1 is disposed to be in contact with the first contact area S1 and the second contact area S2, the guide wire W1 can be held while the longitudinal direction of the sheath 10 and the extension direction of the guide wire W1 are substantially parallel. Thus, even when being used in the rendezvous method, the medical instrument 1 can be easily introduced from the duodenal papilla Dp by pulling the guide wire W1. Therefore, the medical instrument 1 can be easily inserted from the duodenal papilla Dp toward the bile duct Bd even when the duodenal papilla Dp is stenosed or occluded. Further, the medical instrument 1 can be also smoothly introduced even when the duodenal papilla Dp is not stenosed.

Further, by using the sheath 10 of which the distal end is obliquely cut, the first contact area S1 and the second contact area S2 can be formed with a simply machining process, the guide wire Wb and the center axis O can form the acute angle, and the angle α can also be easily adjusted.

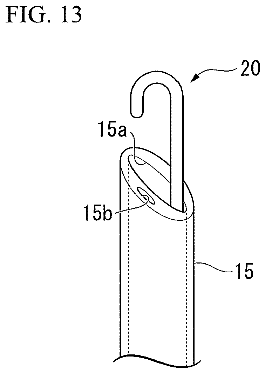

In addition, a configuration using a sheath 15 including a first lumen 15a and a second lumen 15b like a modified example shown in Fig. 13 may be used in the medical instrument . The first lumen 15a and the second lumen 15b are disposed in parallel in the longitudinal direction.

Due to such a configuration, the hook member 20 can be inserted into the first lumen 15a, and a contrast medium can be flowed into the second lumen 15b. Therefore, a state of the bile duct can be checked by flowing the contrast medium into the bile duct when the medical instrument is introduced into the bile duct. Also, the guide wire W2 may be inserted into the second lumen 15b. Since the hook member 20 and the guide wire W2 are disposed in different lumens, interference therebetween does not occur.

Further, the first contact area S1 and the second contact area S2 have been provided at the positions at which phases thereof are deviated by 180°, but the present invention is not limited thereto. When the second contact area is disposed on the proximal end side of the sheath beyond the first contact area, the same effect can be obtained. However, when the phases are deviated by 180°, there is an advantage that the guide wire W can be easily held because the guide wire Wb passes through the center axis O.

Also, a pre-curve (a bending tendency) may be applied such that a surface of the sheath 10 including the second contact area S2 is on an inner side. In such a configuration, since the sheath 10 easily follows the guide wire W, the sheath is easily inserted into the duodenal papilla Dp. Furthermore, the sheath 10 is easily bent by applying the same bending tendency as that of the sheath 10 to the hook member 20.

### [Second comparative example]

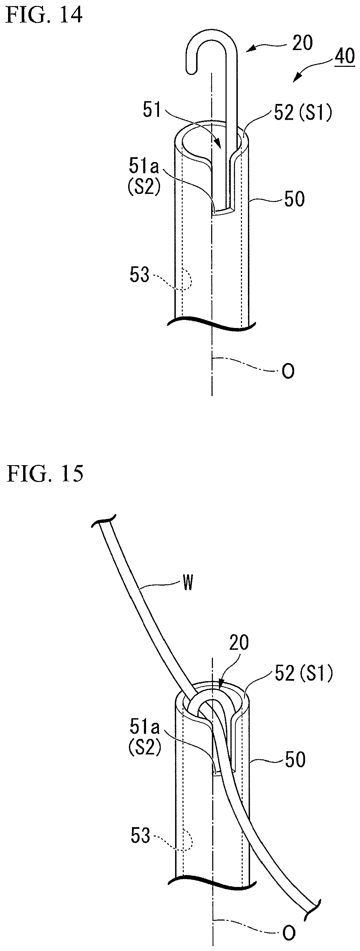

A second comparative example will be described with reference to Figs. 14 to 18.

A medical instrument 40 of the present comparative example is different from that of the first comparative example in a configuration of the sheath.

In the following description, the same reference numerals will be given to the same components as those of the above description, and a repetitive description thereof will be omitted.

As shown in Fig. 14, a sheath 50 has a substantially tubular shape, and a cut-out portion 51 is formed in a distal end thereof to be cut out into a rectangular shape substantially parallel to a center axis O. In the medical instrument 40, the first contact area S1 is a distal end surface 52 of the sheath 50, and the second contact area S2 is a bottom surface 51a of the cut-out portion 51.

A dimension of the cut-out portion 51 in a direction perpendicular to the center axis O is formed larger than that of an outer diameter of the guide wire W. Therefore, the guide wire W can be inserted into the cut-out portion 51.

The hook member 20 is disposed in a lumen 53 of the sheath 50 to be advanceable and retractable in a direction parallel to the center axis O.

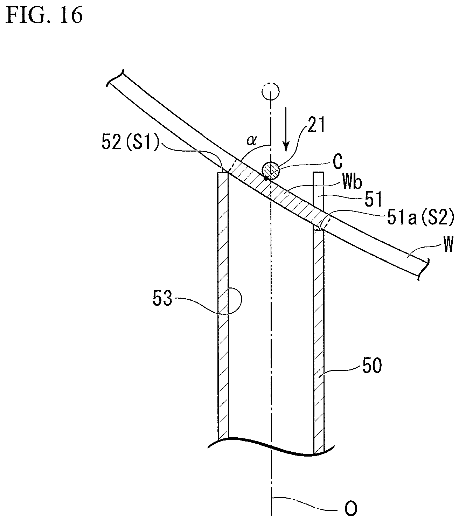

As shown in Fig. 15, when the hook member 20 is slightly retracted while the guide wire W is hooked with the hook portion 21, the guide wire W is pressed to the distal end surface of the sheath 10. As a result, the guide wire W is disposed to be in contact with the first contact area S1 disposed at the distal end surface 52 of the sheath and the second contact area S2 disposed at the bottom surface 51a of the cut-out portion 51. At this time, as shown in the cross section in Fig. 16, the center axis O and the guide wire Wb between the first contact area S1 and the second contact area S2 form an acute angle at a side of the first contact area S1. That is, an angle α formed between the center axis O and the wire Wb located on the distal end side beyond a contact area C at which the hook portion 21 and the wire Wb are in contact with each other is an acute angle.

When the medical instrument 40 is introduced into a bile duct, a method in the present comparative example is different from the method described in the first comparative example in only the fourth step.

In the present comparative example, in the fourth step, the guide wire W is hooked with the hook portion 21 of the medical instrument 40 while the guide wire W protruding from the duodenal papilla Dp is checked with an endoscopic image. Then, as shown in Fig. 16, when the hook member 20 is retracted, the guide wire W is pulled such that the guide wire W1 at a distal end side is brought into contact with the first contact area S1 and the guide wire W1 at a proximal end side is brought into contact with the second contact area S2. Accordingly, the angle α formed between the center axis O and the wire Wb located on the distal end side beyond the contact portion C at which the hook portion 21 and the guide wire Wb are in contact with each other becomes an acute angle.

According to the medical instrument 40 of the present comparative example, since the second contact area S2 is disposed at the bottom surface 51a of the cut-out portion 51, the guide wire W can be held without positional deviation from the second contact area S2.

Further, since the cut-out portion 51 is cut out toward the proximal end side of the sheath 50, it is easy to provide the first contact area S1 on the distal end side beyond the second contact area S2. Therefore, the angle α formed between the center axis O and the wire Wb located on the distal end side beyond the contact portion C at which the hook portion 21 and the wire Wb are in contact with each other easily becomes an acute angle.

In addition, in the present comparative example, only the second contact area S2 is disposed at the bottom surface 51a of the cut-out portion 51, but the present invention is not limited thereto. That is, the cut-out portion may be formed at a position at which a phase thereof is deviated from the second contact area S2 by 180° when being seen from a direction of the center axis O, and the first contact area S1 may be disposed at the bottom surface of the cut-out portion. In such a configuration, the bottom surface of the cut-out portion at a side of the first contact area S1 may be formed on the distal end side compared to the bottom surface of the cut-out portion at a side of the second contact area S2.

Also, the cut-out portion of the present comparative example may be formed in the sheath 10 of the first comparative example. That is, at least one of the first contact area and the second contact area of the sheath 10 of the first comparative example may be formed at the bottom surface of the cut-out portion. Such a configuration can further prevent the positional deviation of the guide wire W, compared to the first comparative example. Further, the angle α can be adjusted without changing the shape of the distal end surface.

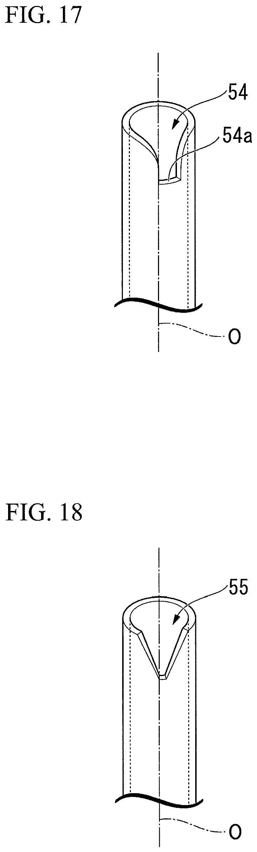

In addition, in the present comparative example, the cut-out portion 51 has been formed in the rectangular shape, but the present invention is not limited thereto. The cut-out portion 51 may be formed in a trapezoidal shape or a triangular shape which has a long side at the distal end side of the sheath 50. For example, as shown in Fig. 17, a cut-out portion 54 of which a cut-out dimension in a direction perpendicular to the center axis O is gradually reduced toward a bottom surface 54a may be used. Like this, since the distal end side of the sheath 50 is widely cut out, the guide wire W1 is easily disposed in the cut-out portion 51 when the guide wire W1 is pulled with the hook member 20.

Further, as shown in Fig. 18, a V-shaped cut-out portion 55 of which a cut-out dimension in a direction perpendicular to the center axis O is gradually reduced toward the proximal end side of the sheath 50 may be used. As described above, the positional deviation of the guide wire W can be prevented by narrowing the bottom surface 51a of the cut-out portion 51 or not providing the bottom surface.

### [Embodiment]

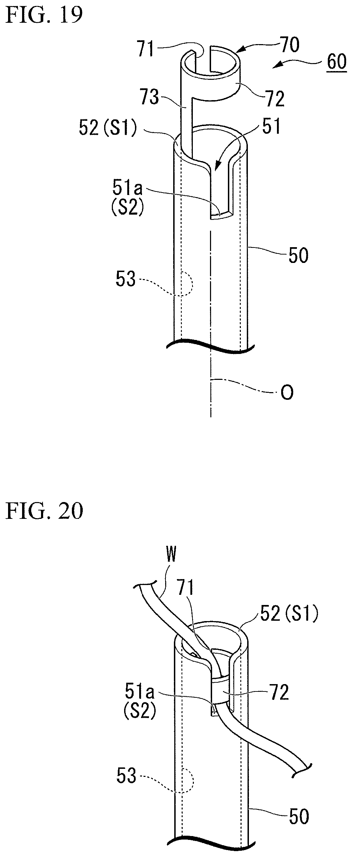

An embodiment of the present invention will be described with reference to Figs. 19 and 20.

A medical instrument 60 of the present embodiment is different from that of the second comparative example in a configuration of the holding portion.

In the medical instrument 60, a holding portion 70 is disposed in the lumen 53 of the sheath 50 to be advanceable and retractable in a direction parallel to the center axis O. The holding portion 70 includes an annular portion 72 having a slit portion 71 through which the guide wire W can pass and a plate-shaped portion 73 which extends in a direction of the center axis O and is connected to the annular portion 72.

A dimension of the slit portion 71 in a direction perpendicular to the center axis O is formed larger than that of an outer diameter of the guide wire W. Therefore, the guide wire W can pass through the slit portion 71.

As shown in Fig. 20, the guide wire W is hooked by the slit portion 71 and disposed such that the guide wire W is in contact with the first contact area S1 and the second contact area S2. At this time, the guide wire W which is in contact with the second contact area S2 is held by the bottom surface 51a of the cut-out portion 51 and the annular portion 72. However, the annular portion 72 and the guide wire W are not in contact with each other at the first contact area S1, and the guide wire W is supported by the first contact area S1.

According to the medical instrument 60 of the present embodiment, since the guide wire W in contact with the second contact area S2 can be held such that the guide wire W is interposed between the bottom surface 51a of the cut-out portion 51 and the annular portion 72, a position of the guide wire W in contact with the second contact area S can be fixed. That is, since it is difficult for the guide wire W to escape from the cut-out portion 51, it is easier to introduce the medical instrument 60 from a duodenal papilla Dp.

Further, the sheath 50 of the second comparative example is used in the present embodiment. However, the holding portion 70 of the present embodiment may be used in the sheath 10 of the first comparative example.

While a preferred embodiment of the present invention has been described above, the present invention is not limited to the embodiment. The invention is defined solely by the appended claims.

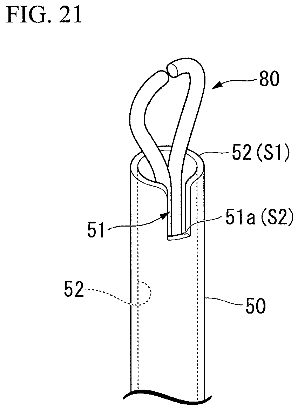

According to a third comparative example, as shown in Fig. 21, a two-legged forceps 80 having one pair of grasping portions of which distal ends are disposed to be spaced apart from each other may be used as the holding portion.

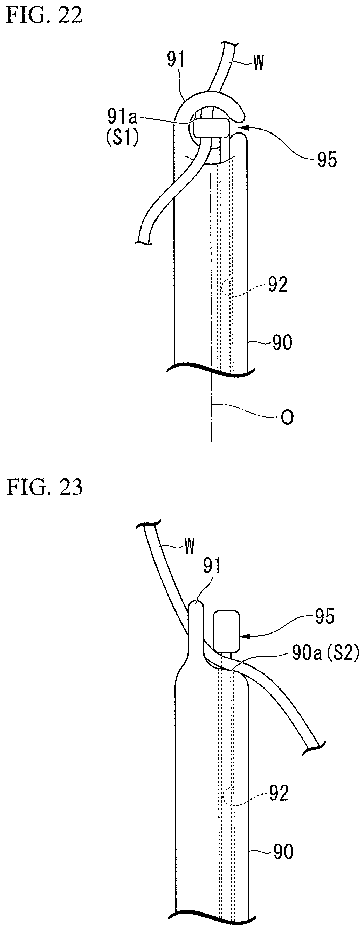

Also, according to a fourth comparative example, as shown in Fig. 22, a sheath 90 at which a hook potion 91 protruding toward a distal end thereof is formed may also be used. A lumen 92 is formed at the sheath 90. A holding portion 95 is disposed to be advanceable and retractable in the lumen 92. An external dimension of a distal end of the holding portion 95 is larger than an inner diameter of the lumen 92. A shape of a distal end portion of a holding portion in a modified example may be the hook portion 21 of the above-described hook member 20.

In the modified example, the first contact area S1 is an inner surface 91a of the hook portion 91, and the second contact area S2 is a distal end surface 90a of the sheath 90 as shown in Fig. 23.

Even in such a configuration, the guide wire W is held by a distal end portion of the holding portion 95 such that the guide wire W is brought into contact with the first contact area S1 and the second contact area S2, and thus the center axis O and the guide wire Wb between the first contact area S1 and the second contact area S2 form an acute angle at a side of the first contact area S1. Accordingly, it is easy to introduce the medical instrument from a duodenal papilla Dp.

### [Additional Note]

The present disclosure includes the following technical idea.

A exemplary method of inserting a medical instrument, which includes a sheath having a center axis, a first contact area capable of being in contact with the guide wire, and a second contact area capable of being in contact with the guide wire and provided on a proximal end side beyond the first contact area, and a holding portion disposed to be advanceable and retractable in a lumen of the sheath and holding the guide wire, into a luminal organ includes: a step of inserting a distal end of the guide wire into a first luminal organ from an outside of a body; a step of causing the guide wire inserted into the first luminal organ to protrude from an opening portion of the first luminal organ into a second luminal organ communicating with the first luminal organ via the opening portion of the first luminal organ and placing a distal end part of the guide wire in the second luminal organ; a step of inserting an endoscope to the second luminal organ, inserting the medical instrument through a treatment tool channel of the endoscope, and causing the medical instrument to protrude from the endoscope; a step of holding the guide wire with the holding portion of the medical instrument such that the guide wire is in contact with the first contact area and the second contact area; and a step of pulling the guide wire to the outside of the body so that a distal end portion of the medical instrument is pulled from the second luminal organ into the first luminal organ via the opening portion.

The present invention is not limited by the description above and is limited only by the appended claims.

### Industrial Applicability

The medical instrument of the embodiment (including modified examples) can be appropriately introduced into a luminal organ by the rendezvous method.

### Reference Signs List

- O:: center axis
- S1:: first contact area
- S2:: second contact area
- W:: guide wire (wire)
- 1, 40, 60:: medical instrument
- 10, 50, 90:: sheath
- 12, 53:: lumen
- 20:: hook member (holding portion)
- 51:: cut-out portion
- 70, 95:: holding portion
- 71:: slit portion
- 72:: annular portion
- 73:: plate-shaped portion
- 80:: two-legged forceps

## Claims

1. A medical instrument (60) which is capable of holding a guide wire (W) protruding from a first luminal organ (Bd) into a second luminal organ (D) via an opening portion (Dp), comprising:
a sheath (50) which has a center axis (O) extending in a longitudinal direction from a distal end thereof toward a proximal end thereof and which includes a first contact area (S1), which is formed on the most distal end side of a distal end surface (52) of the sheath (50) and which is capable of being in contact with the guide wire (W), and a second contact area (S2), which is formed on the most proximal end side of the distal end surface (52) of the sheath (50) and which is capable of coming into contact with the guide wire (W); and
a holding portion (70) which is advanceable and retractable in a lumen (53) of the sheath (50) and is retracted into the lumen (53) of the sheath (50) so that the guide wire (W) is pressed to the first and second contact areas (S1, S2) and the holding portion (70) holds the guide wire (W),
wherein the distal end surface (52), formed at the distal end of the sheath (50), is an inclined surface which is formed in an elliptical shape and is inclined with respect to the center axis (O); and
wherein the guide wire (W) is held by the sheath (50) at two places corresponding to the first contact area (S1) and the second contact area (S2), and
**characterized in that**
the holding portion (70) has an annular portion (72) having a slit portion (71) in which the guide wire (W) is capable of being inserted, and a plate-shaped portion (73) which extends in a direction of the center axis (O) and is connected to the annular portion (72).

2. The medical instrument (60) according to claim 1, wherein the holding portion (70) is capable of holding the guide wire (W) on a straight line connecting the first contact area (S1) and the second contact area (S2) when the sheath (50) is seen from a center axis (O) direction thereof.

3. The medical instrument (60) according to claim 1,
wherein a cut-out portion (51) which is cut out along the center axis (O) is formed in the distal end of the sheath (50), and at least one of the first contact area (S1) and the second contact area (S2) is provided at a bottom surface (51a) of the cut-out portion (51).

4. The medical instrument (60) according to claim 3, wherein
the first contact area (S1) is provided at the most distal end side of the distal end surface (52) of the sheath (50), and
the second contact area (S2) is provided at the bottom surface (51a) of the cut-out portion (51).

## Patentansprüche

1. Medizinisches Instrument (60), das dazu in der Lage ist, einen Führungsdraht (W) zu halten, der über einen Öffnungsabschnitt (Dp) von einem ersten luminalen Organ (Bd) in ein zweites luminales Organ (D) vorsteht, und das umfasst:
eine Hülse (50), die eine Mittelachse (O) aufweist, welche sich in einer Längsrichtung von ihrem distalen Ende zu ihrem proximalen Ende erstreckt, und die einen ersten Kontaktbereich (S1), der auf der distalsten Endseite einer distalen Endfläche (52) der Hülse (50) ausgebildet ist und der dazu in der Lage ist, in Kontakt mit dem Führungsdraht (W) zu sein, und einen zweiten Kontaktbereich (S2), der auf der proximalsten Endseite der distalen Endfläche (52) der Hülse (50) ausgebildet ist und der dazu in der Lage ist, in Kontakt mit dem Führungsdraht (W) zu geraten, umfasst; und
einen Halteabschnitt (70), der in einem Lumen (53) der Hülse (50) vorschiebbar und zurückziehbar ist und in das Lumen (53) der Hülse (50) zurückgezogen wird, so dass der Führungsdraht (W) an den ersten und den zweiten Kontaktbereich (S1, S2) gedrückt wird und der Halteabschnitt (70) den Führungsdraht (W) hält,
wobei die distale Endfläche (52), die an dem distalen Ende der Hülse (50) ausgebildet ist, eine geneigte Fläche ist, die in einer elliptischen Form ausgebildet ist und in Bezug auf die Mittelachse (O) geneigt ist; und
wobei der Führungsdraht (W) durch die Hülse (50) an zwei Stellen gehalten wird, die dem ersten Kontaktbereich (S1) und dem zweiten Kontaktbereich (S2) entsprechen, und
**dadurch gekennzeichnet, dass**
der Halteabschnitt (70) einen ringförmigen Abschnitt (72) mit einem Schlitzabschnitt (71), in den der Führungsdraht (W) einführbar ist, und einen plattenförmigen Abschnitt (73) aufweist, der sich in einer Richtung der Mittelachse (O) erstreckt und mit dem ringförmigen Abschnitt (72) verbunden ist.

2. Medizinisches Instrument (60) nach Anspruch 1, wobei der Halteabschnitt (70) dazu in der Lage ist, den Führungsdraht (W) auf einer geraden Linie zu halten, die den ersten Kontaktbereich (S1) und den zweiten Kontaktbereich (S2) verbindet, wenn die Hülse (50) aus der Richtung ihrer Mittelachse (O) betrachtet wird.

3. Medizinisches Instrument (60) nach Anspruch 1,
wobei ein ausgeschnittener Abschnitt (51), der entlang der Mittelachse (O) ausgeschnitten ist, in dem distalen Ende der Hülse (50) ausgebildet ist, und der erste Kontaktbereich (S1) und/oder der zweite Kontaktbereich (S2) an einer Bodenfläche (51a) des ausgeschnittenen Abschnitts (51) vorgesehen ist.

4. Medizinisches Instrument (60) nach Anspruch3, wobei
der erste Kontaktbereich (S1) an der distalsten Endseite der distalen Endfläche (52) der Hülse (50) vorgesehen ist, und
der zweite Kontaktbereich (S2) an der Bodenfläche (51a) des ausgeschnittenen Abschnitts (51) vorgesehen ist.

## Revendications

1. Instrument médical (60) qui est apte à maintenir un fil guide (W) faisant saillie depuis un premier organe luminal (Bd) jusque dans un deuxième organe luminal (D) par l'intermédiaire d'une partie d'ouverture (Dp), comprenant :
une gaine (50) qui a un axe central (O) s'étendant dans un sens longitudinal d'une extrémité distale de celle-ci vers une extrémité proximale de celle-ci et qui inclut une première zone de contact (S1), qui est formée sur le côté d'extrémité le plus distal d'une surface (52) d'extrémité distale de la gaine (50) et qui est apte à être en contact avec le fil guide (W), et une deuxième zone de contact (S2), qui est formée sur le côté d'extrémité le plus proximal de la surface (52) d'extrémité distale de la gaine (50) et qui est apte à venir en contact avec le fil guide (W) ; et
une partie (70) de maintien qui peut être avancée et rétractée dans une lumière (53) de la gaine (50) et est rétractée dans la lumière (53) de la gaine (50) de telle sorte que le fil guide (W) est pressé sur les première et deuxième zones de contact (S1, S2) et la partie (70) de maintien maintient le fil guide (W),
dans lequel la surface (52) d'extrémité distale, formée à l'extrémité distale de la gaine (50), est une surface inclinée qui est formée à une forme elliptique et est inclinée par rapport à l'axe central (O) ; et
dans lequel le fil guide (W) est maintenu par la gaine (50) en deux endroits correspondant à la première zone de contact (S1) et à la deuxième zone de contact (S2), et
**caractérisé en ce que**
la partie (70) de maintien a une partie annulaire (72) ayant une partie (71) de fente dans laquelle le fil guide (W) est apte à être inséré, et une partie (73) en forme de plaque qui s'étend dans un sens de l'axe central (O) et est connectée à la partie annulaire (72).

2. Instrument médical (60) selon la revendication 1, dans lequel la partie (70) de maintien est apte à maintenir le fil guide (W) sur une ligne droite connectant la première zone de contact (S1) et la deuxième zone de contact (S2) lorsque la gaine (50) est vue depuis un sens d'axe central (O) de celle-ci.

3. Instrument médical (60) selon la revendication 1,
dans lequel une partie découpée (51) qui est découpée le long de l'axe central (O) est formée dans l'extrémité distale de la gaine (50), et au moins une parmi la première zone de contact (S1) et la deuxième zone de contact (S2) est prévue au niveau d'une surface de fond (51a) de la partie découpée (51).

4. Instrument médical (60) selon la revendication 3, dans lequel
la première zone de contact (S1) est prévue au niveau du côté d'extrémité le plus distal de la surface (52) d'extrémité distale de la gaine (50), et
la deuxième zone de contact (S2) est prévue au niveau de la surface de fond (51a) de la partie découpée (51).
